# EUROPEAN PATENT SPECIFICATION

(11) **EP 1 051 961 B1**
(45) Date of publication and mention of the grant of the patent: **22.02.2006**
(21) Application number: 00109717.9
(22) Date of filing: 08.05.2000
(51) Int. Cl.: A61K 6/083

(54) **Bonding compositions for dental use**
Klebemassen für den Dentalbereich
Compositions adhésives à usage dentaire

(30) Priority: 13.05.1999 JP 13208599
(43) Date of publication of application: 15.11.2000
(73) Proprietor: KURARAY CO., LTD., Kurashiki-shi, Okayama 710-8622 (JP)
(72) Inventor: Hino, Kenichi, c/o Kuraray Co., Ltd., Okayama 710-8622 (JP)
(74) Representative: Müller-Boré & Partner Patentanwälte

(56) References cited:
- EP-A- 0 835 646
- EP-A- 1 123 691
- WO-A-93/12760
- GB-A- 2 332 911
- US-A- 4 719 149
- US-A- 5 264 513

## Description

The present invention relates to a bonding composition, especially to a self-etching and self-priming bonding composition for dental use for restoring defective teeth. When used in dental treatment for restoring a defective tooth with a restorative dental material, the bonding composition does not require any pre-treatment including acid etching or priming treatment, and keeps the restorative dental material bonded to the defective tooth for a long period of time.

In the last half of this century, the technology of bonding an acrylic resin to teeth has a great influence on the development of dental treatment. For its history, first referred to is the technique developed by M. Buonocore in 1956. This comprises etching and roughening the enamel of a tooth with an aqueous solution of phosphoric acid so as to significantly increase the area of the bonding interface between the tooth and the resin applied thereto, thereby increasing the bonding strength between them. After this, various types of functional monomers have been developed, which are for forming chemical bonds to the components of teeth. With those, the technique of bonding resins to the dentin of teeth has made remarkable progress in dental treatment. The functional monomers proposed include, for example, polymerizable monomers having an acid group such as a phosphoric acid residue, a carboxylic acid residue or the like with high reactivity to the essential ingredient, calcium hydroxyapatite (HAP) of teeth; and other monomers capable of forming covalent bonds to the collagen of teeth.

Of those, one remarkable technique is to add water to a dental bonding composition that contains a phosphoric acid residue-having monomer. This was proposed by the present applicant. Concretely, it is an invention relating to a dental bonding composition that comprises, as the constituent ingredients, a specific polymerizable monomer having a phosphoric acid residue in the molecule, a specific terpolymerization initiator selected from diacyl peroxides, amine compounds, arylsulfinic acid salts and others, water, and optionally any other copolymerizable monomer (see Japanese Patent Laid-Open No. 45510/1985). Contrary to the conventional knowledge of such that how to remove the influence of water on the restored area of teeth is the important theme for ensuring good adhesion of a restorative material to teeth that are all the time kept in contact with saliva, the invention already proposed is based on the finding that adding water to the dental bonding composition to be one ingredient thereof enhances the bonding strength of the composition. The inventors say in the specification of 45510/1985 that the dental bonding composition could exhibit high bonding strength even to the teeth not previously etched with an acid, but its bonding strength could be enhanced more when the teeth to which it is applied are previously etched with an acid in an ordinary manner before the composition is applied thereto. However, they did not intend to provide a self-etching or self-priming, dental bonding composition which is self-etchable or self-primable by itself.

After that, it was found that primers, if applied to teeth before bonding compositions thereto, could enhance the bonding strength of the compositions to a great extent, and various proposals have been made for primers. Based on the intrinsic function of ordinary bonding compositions that are "to wet the objects to which they are applied and are compatible with the objects", most of the proposals would be to make the objects surely wetted by the bonding compositions applied thereto. For example, proposed was an invention relating to a primer for hard tissue, which comprises an acid, a water-soluble film-forming agent, and optionally water, as in Japanese Patent Laid-Open No. 223289/1987. A specific acid is used in the invention proposed, and its salt with calcium must be soluble in the water-soluble film-forming agent (substantially in water, as in the illustrated Examples of the invention) in order that the primer could ensure high bonding strength. The specification of the invention says that, when the primer is first applied to a tooth and thereafter a bonding composition is applied thereto, then the initial bonding strength of the bonding composition to the dentin of the tooth is increased. However, when the bonded tooth sample is kept in water for a long period of time, the bonding strength of the bonding composition decreases, and the bonding durability thereof is not satisfactory. Thus, the primer proposed is problematic in this respect.

On the other hand, another invention was proposed, relating to a primer composition that comprises water, a hydroxyl group-having polymerizable compound, an acid group-having polymerizable compound, and a curing agent (see Japanese Patent Laid-Open No. 240712/1991). The primer proposed indispensably comprises the four ingredients including a curing agent as above, and its object is to ensure high bonding strength and bonding durability. In this, however, the acid group-having polymerizable compound (that is, an acid) is not specifically defined, and includes the acid group-having polymerizable compounds that are exemplified as acids in the specification of the above-mentioned 223289/1987.

The two techniques of using the primers proposed are to employ the primer treatment of teeth in place of the ordinary etching treatment thereof with phosphoric acid. These could ensure high bonding strength, but indispensably requires bonding treatment with a liner or the like bonding composition after the primer treatment except for resin cement and the like that is adhesive by itself. In this respect, therefore, the techniques are still problematic in that the primers proposed are not to shorten the process of dental treatment with them and could not fully meet the requirement of the users, dentists and others.

WO 93/12760 discloses a method for adhering to or coating hard tissue, comprising the steps of applying to said hard tissue adhesively effective amounts of an acid and a water-dispersible film former and hardening said film former.

EP-A-0 835 646 discloses an adhesive composition for dental or surgical treatment comprising an organic boron compound, a photopolymerization initiator and a polymerizable monomer mixture.

US-A-5,264,513 discloses a primer composition which comprises water, a compound having a hydroxyl group and a polymerizable unsaturated group, a compound having an acidic group and a polymerizable unsaturated group and a curing agent.

US-A-4,719,149 discloses a method for adhering to or coating dentin, comprising the steps of applying to said dentin adhesively effective amounts of an acid and water-soluble film former and hardening said film former.

GB-A-2,332,911 discloses a dental adhesive kit comprising a combination of a self etching primer and a bonding agent.

EP-A-1 123 691 describes the use of a composition for the preparation of a dental restorative composition for binding to hard tooth, metal and ceramic.

In that situation, one object of the present invention is to provide a self-etching and self-priming bonding composition which has the function of acid etching and priming effect by itself and ensures good initial bonding strength to teeth and long-lasting bonding durability thereto. Not requiring any pre-treatment including acid etching treatment and priming treatment, the self-etching and self-priming bonding composition is especially favorable to dental treatment and ensures good bonding to tooth tissue.

Another object of the invention is to provide a one-liquid bonding composition having the advantage of rapid curability.

Still another object of the invention is to provide a bonding composition having the advantage of increased bonding strength of the layer formed of it. Another advantage of the bonding composition is that the thickness of its layer can be controlled in any desired manner.

I, the present inventor have studied the mechanism of the bonding behavior and the bonding durability of the bonding compositions in the prior art noted above, and have reached the following conclusion: The dentin of teeth is a type of complicated bio-tissue that comprises collagen fibers and HAP crystals. When an acidic primer is applied thereto, its acid ingredient will partially dissolve the HAP crystals in the dentin (for this, it is believed that the acid ingredient of the primer applied to a tooth would etch the tooth, and the primer could thereby enjoy the self-etching function), and, at the same time, the water-soluble monomer ingredient of the primer will penetrate into the collagen fibers having remained in the dentin after the HAP crystals were dissolved away. As a result, the surface of the dentin thus having been processed with the primer will be so modified that it is well compatible with a bonding agent. In that condition, the primer is then cured on the dentin whereby the collagen fibers of the dentin will be entangled together with the resin as formed through polymerization of the monomers and the water-soluble film-forming agent constituting the bonding composition applied to the tooth after the primer thereto. As a result, the bonding composition could have good bonding strength to the tooth.

The boundary area between the dentin of the tooth and the resin layer formed thereon from the primer is analyzed. In the boundary area between them, a calcium salt of the acid having formed through the reaction of the acid with HAP exists around the surface of the HAP crystals. In addition, resin portions containing the collagen fibers as left as they are after the partial dissolution of HAP, and also the calcium salt of the acid shall exist in that boundary. Accordingly, in case where the calcium salt of the acid is soluble in water and when the bonded sample is kept in water for a long period of time, the calcium salt in the resin existing around the bonding interface will gradually dissolve out in water whereby the packing density of the bonding resin around the bonding interface will be lowered. As a result, the strength of the bonding resin will be lowered and the bonding durability of the bonding layer will be lost.

I, the present inventor have specifically noted the advantage of the prior art primers capable of expressing high bonding strength even when they are directly applied to teeth not previously subjected to acid etching treatment (this will probably the self-etching effect of the primers), and the disadvantage thereof not having good bonding durability because the calcium salt of the acid existing in the bonding interface layer is soluble in water, and have assiduously studied to overcome the drawback of the prior art primers on the basis of the fundamental knowledge relating to the composition of ordinary bonding compositions for dental use. As a result, we have reached the following knowledge:
(1) As the acid to be in bonding compositions for dental use, those having a specific acid strength are preferred. More preferred are those having a polymerizable group in the molecule and capable of forming a stable chemical bond to the HAP and amino group of collagen existing in teeth structure and also a stable chemical bond even to the water-soluble polymerizable compound to be coated over teeth. Selectively using the specific acid in a bonding composition ensures better bonding durability of the composition.
(2) As the acid, preferred are those of which the calcium salts to be formed as a result of acid etching with them have a lower degree of solubility in water. This is because the calcium salt of the acid existing around the bonding interface does not dissolve in water, and therefore the strength of the bonding resin could be prevented from being lowered.
(3) As the film-forming agent, preferred are those that are soluble in the acid used to any desired degree and are copolymerizable with the acid. More preferred are those miscible with the fluid in body tissue, as they could well penetrate into hard tissue.
(4) Preferably, a polymerization initiator is added to bonding compositions. More preferably, polyfunctional monomers and others are added thereto within the range not interfering with the uniformity of the compositions. These are to promote the polymerization of the bonding layer and to make the polymerized bonding layer more harder with introducing a crosslinked structure, whereby the bonding strength of the bonding layer at the bonding interface is further enhanced and the bonding durability thereof could be also enhanced.
(5) A form-retaining agent is added to bonding compositions. It reinforces the bonding layer and, in addition, ensures the desired thickness of the bonding layer. Accordingly, the polymerization failure in the bonding interface that may be caused by oxygen in air could be prevented, and the bonding interface could be smoothly polymerized and cured to ensure increased bonding strength of the cured bonding layer.

Based on my studies and the results as above, I prepared samples of a bonding composition to be mentioned below, and tested them for their bonding behavior. Precisely, we applied the samples to the dentin of bovine teeth and cured them thereon. Immediately after curing, the bonding layer had a bonding strength of at least 15 MPa. After the bonded samples were stored in water at 50°C for one month, the bonding strength of the bonding layer was still at least 14 MPa. After the bonding test, the samples were observed, and most of them showed cohesive failure with the bonded tooth broken. The numerical data we obtained are as above, but it is believed that the actual bonding strength of the bonding composition should be higher than the level of the data. On the basis of these findings, we have reached and completed the present invention.

Specifically, the invention is a bonding composition suitable to tooth tissue, which comprises a mixture of a (meth)acryloyloxyalkyl phosphate in which the number of the carbon atoms consisting the alkyl group falls between 6 and 24 , a water-soluble film-forming agent, water, and a curing agent, and in which the calcium salt of the acid is insoluble in water, and the film-forming agent is a polymerizable compound and is miscible with a physiological saline solution. The bonding composition contains a form-retaining agent, selected from the group consisting of sand balloons, glass balloons, short glass fibers, pieces of hollow glass fibers, glass beads, glass powders, powders of natural minerals, beads and flakes of crosslinked polymers and organic/inorganic composite materials containing said inorganic substances or crosslinked polymers.

The (meth)acryloyloxyalkyl phosphate to be in the bonding composition of the invention as the acid may be soluble in water, or may dissolve in water to form micelles therein. For ensuring good bonding behavior of the composition, the (meth)acryloyloxyalkyl phosphate is preferably as follows: When it is mixed in water to have a concentration of 1 % by weight, the pH of the resulting mixture is at most 3, preferably at most 2.5, more preferably falls between 1.8 and 2.5.

The calcium salt of the (meth)acryloyloxyalkyl phosphate must be insoluble in water, and it is substantially insoluble also in the film-forming agent. The solubility of the calcium salt of the phosphate, i.e. the acid, may be checked as follows: A predetermined amount of calcium carbonate is added to a plurality of solutions of an acid in water having varying acid concentrations, or to a plurality of solutions of the acid in a film-forming agent having varying acid concentrations, so that a stoichiometrically 1.05 equivalent times of the acid is reacted with calcium carbonate to form a calcium salt of the acid in the reaction solution. Where the reaction solution is clear, it is recognized that the calcium salt of the acid formed dissolved in the solution; but where it is cloudy, it is recognized that the calcium salt of the acid formed did not dissolve therein. When the solubility in water of the calcium salt measured in this method falls between 0.0001 and 0.05 mols/liter (M/L), then the calcium salt is insoluble in water, and satisfies the requirement of the invention. Preferably, the solubility in water of the calcium salt of the acid for use in the invention falls between 0.0001 and 0.01 M/L, more preferably between 0.0001 and 0.001 M/L.

For the insoluble calcium salt, preferred are those of acids that are soluble in water in some degree in the initial reaction stage just after their formation through reaction with teeth but are gradually converted into more stable insoluble salts in the reaction system, to those of acids such as sulfuric acid and the like that may be readily formed immediately after contacted with teeth. Preferably, the calcium salt of the acid is soluble in some degree in the acid-containing bonding composition, but after the acid in the composition has completely reacted with a tooth, the resulting calcium salt is not soluble in the composition not containing the acid. If hydrochloric acid, nitric acid, p-toluenesulfonic acid or the like is used in place of the (meth)acryloyloxyalkyl phosphate in the bonding composition, the solubility in water of the calcium salt of the acid is too high and the composition could not exhibit high bonding strength and good bonding durability. Therefore, these acids are unsuitable to the invention. On the other hand, the solubility in water of the calcium salts of phosphoric acid, oxalic acid or the like, if used, is too low; and the calcium salt of sulfuric acid, if used, crystallizes too rapidly. Therefore, the bonding composition containing any of these acids could not be self-etchable to a satisfactory degree, and the acids are unsuitable to the invention.

The (meth)acryloyloxyalkyl phosphate for use in the invention is not specifically defined, so far as it satisfies the requirement mentioned above. Examples of (meth)acryloyloxyalkyl phosphates (this expression means acryloyloxyalkyl phosphates and methacryloyloxyalkyl phosphates, and the same shall apply hereunder) in which the number of the carbon atoms constituting the alkyl group falls between 6 and 24, are 6-(meth)acryloyloxyhexyl phosphate, 8-(meth)acryloyloxyoctyl phosphate, 10-(meth)acryloyloxydecyl phosphate, 12-(meth)acryloyloxylauryl phosphate, 16-(meth)acryloyloxycetyl phosphate, 18-(meth)acryloyloxystearyl phosphate, 20-(meth)acryloyloxyeicosyl phosphate.

One or more of these may be used singly or as combined. These may be further combined with any other acid compounds so far as the combinations could attain the object of the invention. Of these compounds, preferred are (meth)acryloyloxyalkyl phosphates in which the number of the carbon atoms constituting the alkyl group falls between 8 and 20 or so. Especially preferred is 10-methacryloyloxydecyl phosphate (that is, 10-methacryloyloxydecyl dihydrogenphosphate).

The amount of the (meth)acryloyloxyalkyl phosphate to be in the bonding composition of the invention may vary within the range within which the composition could attain the object of the invention. Preferably, however, it falls between 0.1 and 50 % by weight, more preferably between 1 and 50 % by weight, even more preferably between 2 and 40 % by weight of the bonding composition.

The film-forming agent to be in the bonding composition of the invention shall differ from the (meth)acryloyloxyalkyl phosphate mentioned above, and it is a water-soluble polymerizable compound and shall be miscible with a physiological saline solution that may be a substituent for the fluid in body tissue. Even though not fully soluble in water and not miscible with a physiological saline solution by themselves, polymerizable compounds could be used as the water-soluble film-forming agent in the invention, so far as a mixture of two or more of them or a mixture of the polymerizable compound with the (meth)acryloyloxyalkyl phosphate to be in the bonding composition and/or with an organic solvent that will be mentioned hereinunder could be soluble in water and could be miscible with a physiological saline solution. Containing the film-forming agent of that type, the bonding composition could form a uniform solution.

The film-forming agent for use in the invention must be soluble in water. Preferably, at least 5 % by weight or so of the agent could dissolve in water. More preferably, it is soluble in water in any ratio. In addition, the water-soluble film-forming agent must be miscible with a physiological saline solution. Whether the film-forming agent is miscible with a physiological saline solution or not should be judged from its phase diagram. Concretely, however, 50 parts by weight of the film-forming agent or the bonding composition is mixed with 50 parts by weight of a physiological saline solution at room temperature, and the resulting mixture is checked as to whether or not it forms a uniform solution. From it, the miscibility of the film-forming agent in a physiological saline solution could be readily judged in a simplified manner.

The film-forming agent may be properly selected from acrylates, acrylamides, crotonates, cinnamates and others that are soluble in water and miscible with a physiological saline solution. However, preferred are (meth)acrylate monomers as they are easy to polymerize and safe to bodies. More preferred are (meth)acrylate monomers having an increased number of hetero (polar) atoms except carbon and hydrogen in the molecule. More preferably, the (meth)acrylate monomers are such that the value obtained by dividing the sum total of the products of the number of the polar atoms therein and the atomic weight of each polar atom, by the molecular weight of the molecule is at least 0.3.

Examples of the compounds are compounds having a hydrophilic group such as a hydroxyl group, a carbonyl group, an amino group, an ammonium salt residue, a phosphonium salt residue, a sulfonic acid salt residue, an ether bond, a cyclic ether group, an acyl group or the like in the molecule. They include, for example, hydroxyl group-having (meth)acrylates such as 2-hydroxyethyl (meth)acrylate, 2-hydroxypropyl (meth)acrylate 1,3- and 2,3-dihydroxypropyl (meth)acrylates, pentaerythritol mono- and di(meth)acrylates, dipentaerythritol mono-, di- and tri(meth)acrylates, xylitol mono- and di(meth)acrylates, etc.; amides such as (meth)acrylamide, 2-hydroxyethyl(meth)acrylamide, N,N-bis(2-hydroxyethyl)(meth)acrylamide, N-alkyl-N-hydroxyethyl(meth)acrylamides, 2- and 3-hydroxypropyl(meth)acrylamides, methacrylamidopropyltrimethylammonium chloride, etc.; glycol (meth)acrylates such as diethylene glycol mono(meth)acrylate, triethylene glycol mono(meth)acrylate, polyethylene glycol(400) mono(meth)acrylate, methoxypolyethylene glycol (meth)acrylate, etc.; other hydrophilic monomers such as dimethylaminoethyl (meth)acrylate, 2-trimethylammoniumethyl (meth)acrylate hydrochloride, pyrrolidone methacrylate, sorbitol (meth)acrylate, etc. One or more of these may be used either singly or as combined. Of the hydrophilic monomers mentioned above, preferred are hydroxyalkyl (meth)acrylates. Especially preferred is 2-hydroxyethyl methacrylate. Preferably, it is the essential ingredient of the film-forming agent, for example, accounting for at least 50 % by weight of the agent.

In case where two or more monomers are combined to give the film-forming agent, a plurality of the hydrophilic monomers mentioned above will be selected and combined. If desired, any other monomers may be added thereto, so far as they satisfy the requirement that a mixture of 50 parts of the mixed, film-forming agent and 50 parts of a physiological saline solution can form a uniform solution. The additional monomers maybemonofunctional (meth)acrylates, including, for example, methyl (meth)acrylate, ethyl (meth)acrylate, n-butyl (meth)acrylate, isobutyl (meth)acrylate, 2-ethylhexyl (meth)acrylate, lauryl (meth)acrylate, tridecyl (meth)acrylate, stearyl (meth)acrylate, cyclohexyl (meth)acrylate, benzyl (meth)acrylate, glycidyl (meth)acrylate, tetrahydrofurfuryl (meth)acrylate, allyl (meth)acrylate, methoxydiethylene glycol (meth)acrylate, methoxytetraethylene glycol (meth)acrylate, 2-ethoxyethyl (meth)acrylate, phenoxydiethylene glycol (meth)acrylate, phenoxyhexaethylene glycol (meth)acrylate, dicyclopentadiene-dimethanol-di(meth)acrylate, isobornyl (meth)acrylate,phenyl(meth)acrylate, caprolactone-modified tetrahydrofurfuryl (meth)acrylate, caprolactone-modified dipentaerythritol (meth)acrylate, caprolactone-modified 2-hydroxyethyl (meth)acrylate, etc.

Also usable are polyfunctional (meth)acrylates, including, for example, ethylene glycol di(meth)acrylate, diethylene glycol di(meth)acrylate, polyethylene glycol di(meth)acrylate, 2-hydroxypropyl-1,3-di(meth)acrylate, 1,3-butylene glycol di(meth)acrylate, 1,6-hexanediol di(meth)acrylate, neopentylglycol di(meth)acrylate, tripropylene glycol di (meth) acrylate, polypropylene glycol di(meth)acrylate, bisphenol A di(meth)acrylate, bisphenol A-glycidyl di(meth)acrylate, ethylene oxide-modified bisphenol A di(meth)acrylate, ethylene oxide-modified bisphenol A-glycidyl di(meth)acrylate, 2,2-bis(4-methacryloxypropoxyphenyl)propane, 7,7,9-trimethyl-4,13-dioxa-3,14-dioxo-5,12-diazahexadecane-1,16-diol di(meth)acrylate, neopentylglycol hydroxypivalate di(meth)acrylate, caprolactone-modified neopentylglycol hydroxypivalate di(meth)acrylate, trimethylolethane di(meth)acrylate, trimethylolpropane di(meth)acrylate, trimethylolmethane tri(meth)acrylate, trimethylolethane tri(meth)acrylate, trimethylolpropane tri(meth)acrylate, pentaerythritol tri(meth)acrylate, pentaerythritol tetra(meth)acrylate, dipentaerythritol tetra(meth)acrylate, dipentaerythritol hexa(meth)acrylate, reaction product of 3-chloro-2-hydroxypropyl (meth)acrylate and methylcyclohexane diisocyanate, reaction product of 2-hydroxypropyl (meth)acrylate and methylcyclohexane diisocyanate, reaction product of 2-hydroxypropyl (meth)acrylate and methylene bis(4-cyclohexyl isocyanate), reaction product of 2-hydroxypropyl (meth)acrylate and trimethylhexamethylene diisocyanate, reaction product of 2-hydroxyethyl (meth)acrylate and isophorone diisocyanate, reaction product of 3-chloro-2-hydroxypropyl (meth)acrylate and isophorone diisocyanate, etc.

The film-forming agent for use in the invention may be a mixture that comprises, as the essential ingredient, any of the above-mentioned hydrophilic monomers and contains any of the above-mentioned monofunctional (meth)acrylates and/or polyfunctional (meth)acrylates. Preferably, the mixture contains a polyfunctional (meth)acrylate, as ensuring better bonding durability of the bonding layer especially in the bonding interface. In this case, the blend ratio of the monofunctional (meth)acrylate and/or the polyfunctional (meth)acrylate may fall between 0.1 and 40 parts by weight, preferably between 1 and 20 parts by weight to 100 parts by weight of the film-forming agent. The amount of the film-forming agent to be in the bonding composition of the invention may vary within the range capable of attaining the object of the invention. Preferably, it falls between 10 and 98 % by weight, more preferably between 15 and 96 % by weight, even more preferably between 20 and 90 % by weight of the bonding composition.

The bonding composition of the invention must contain water to ensure the bonding ability. Preferably, water is in the products of the composition produced and distributed by manufacturers, but may be added thereto by dentists and other users after they have bought the products or just before they use them. Also preferably, water to be in the bonding composition is pure water such as distilled water, deionized water, water purified through reverse osmosis or the like, but may be tap water when the composition is used in field hospitals, etc. As the case may be, it may be electrically pre-treated in an electric field. The amount of water to be in the bonding composition may vary within the range capable of attaining the object of the invention, but preferably falls between 1 and 80 % by weight, more preferably between 10 and 70 % by weight of the composition.

If desired, a water-soluble, volatile organic solvent may be added to the bonding composition of the invention so as to augment the solvent effect of water in the composition. The organic solvent includes, for example, acetone, methyl ethyl ketone, methanol, ethanol, isopropyl alcohol, etc. One or more of these organic solvents may be used either singly or as combined. Ethanol is especially preferred. The amount of the organic solvent preferably falls between 1 and 50 % by weight, more preferably between 2 and 40 % by weight of the bonding composition.

The bonding composition of the invention must contain a curing agent to ensure the bonding ability of the bonding layer especially in the bonding interface, thereby to augment the bonding strength thereof. The curing agent may be a polymerization initiator generally used in ordinary bonding compositions, or may be a mixture of such a polymerization initiator and a polymerization promoter. The polymerization initiator may be any of photopolymerization initiators, thermal polymerization initiators and their mixtures. Preferably, it is soluble in water. However, if a polymerization initiator poorly soluble in water is used therein, the bonding composition is preferably so conditioned that it does not deposit the initiator on the tooth tissue to which it has been applied or does not precipitate the initiator therein while it is stored.

Preferred photopolymerization initiators are α-diketone compounds, ketal compounds, anthraquinone compounds, thioxanthone compounds, benzoin alkyl ether compounds, acylphosphine oxide compounds, etc.

The α-diketone compounds include, for example, camphorquinone, benzil, diacetyl, acenaphthenequinone, 9,10-phenanthraquinone, etc. The ketal compounds include, for example, benzyldimethyl ketal, benzyldiethyl ketal, benzyldi(β-phenylethyl) ketal, benzyldi(2-methoxyethyl) ketal, etc. The anthraquinone compounds include, for example, anthraquinone, β-methylanthraquinone, β-ethylanthraquinone. The thioxanthone compounds include, for example, 2-ethylthioxanthone, 2-chlorothioxanthone, 2-hydroxy-3-(3,4-dimethyl-9-oxo-9H-thioxanthen-2-yloxy)-N,N,N-trimethyl-1-propaneaminium chloride, etc. The benzoin alkyl ether compounds include, for example, benzoin methyl ether, benzoin ethyl ether, benzoin propyl ether, etc. The acylphosphine oxide compounds include, for example, 2,4,6-trimethylbenzoyldiphenylphosphine oxide, 2,6-dimethylbenzoyldiphenylphosphine oxide, 2,6-dimethoxybenzoyldiphenylphosphine oxide, etc. Of those photopolymerization initiators, especially preferred are camphorquinone, benzil and 2,4,6-trimethylbenzoyldiphenylphosphine oxide.

For thermal polymerization initiators, effectively used are organic peroxides, including, for example, diacyl peroxides, peroxy esters, dialkyl peroxides, peroxy ketals, ketone peroxides, hydroperoxides, etc. The diacyl peroxides include, for example, benzoyl peroxide, 2,4-dichlorobenzoyl peroxide, m-toluoyl peroxide, etc. The peroxy esters includes, for example, t-butylperoxy benzoate, 2,5-dimethyl-2,5-bis(benzoylperoxy)hexane, t-butylperoxy-2-ethylhexanoate, etc. The dialkyl peroxides include, for example, dicumyl peroxide, di-t-butyl peroxide, lauroyl peroxide, etc. The peroxy ketals include, for example, 1,1-bis(t-butylperoxy)-3,3,5-trimethylcyclohexane, etc. The ketone peroxides include, for example, methyl ethyl ketone peroxide, etc. The hydroperoxides include, for example, cumenehydroperoxide, t-butylhydroperoxide , etc. Of those thermal polymerization initiators, especially preferred is benzoyl peroxide.

For the polymerization promoter, preferred are amines, sulfinic acids and their salts, etc. The amines are, for example, aromatic tertiary amines, aliphatic tertiary amines, etc. The aromatic tertiary amines include, for example, N,N-dimethylaniline, N,N-dimethyl-p-toluidine, N,N-diethyl-p-toluidine, N,N-dimethyl-3,5-dimethylaniline, N,N-dimethyl-4-ethylaniline, N,N-dimethyl-4-t-butylaniline, N,N-bis(2-hydroxyethyl)p-toluidine, N,N-bis(2-hydroxyethyl)-3,5-dimethylaniline, N,N-bis(2-hydroxyethyl)-3,4-dimethylaniline, N,N-bis(2-hydroxyethyl)-4-ethylaniline, N,N-bis(2-hydroxyethyl)-4-t-butylaniline, N,N-bis(2-hydroxyethyl)-3,5-di-t-butylaniline, ethyl 4-dimethylaminobenzoate, n-butoxyethyl 4-dimethylaminobenzoate, (2-methacryloyloxy)ethyl 4-dimethylaminobenzoate, 4-dimethylaminobenzophenone, etc. The aliphatic tertiary amines include, for example, trimethylamine, triethylamine, N-methyldiethanolamine, N-ethyldiethanolamine, N-n-butyldiethanolamine, N-lauryldiethanolamine, triethanolamine, (2-dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, etc.

The sulfinic acids and their salts include, for example, benzenesulfinic acid, sodium benzenesulfinate, potassium benzenesulfinate, calcium benzenesulfinate, lithium benzenesulfinate, toluenesulfinic acid, sodium toluenesulfinate, potassium toluenesulfinate, calcium toluenesulfinate, lithium toluenesulfinate, 2,4,6-trimethylbenzenesulfinic acid, sodium 2,4,6-trimethylbenzenesulfinate, potassium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, etc. Of the polymerization promoters mentioned above, preferred are amines such as N,N-bis(2-hydroxyethyl)-p-toluidine, ethyl 4-dimethylaminobenzoate, n-butoxyethyl 4-dimethylaminobenzoate, etc. ; and salts of sulfinic acids such as sodium benzenesulfinate, sodium toluenesulfinate, sodium 2,4,6-triisopropylbenzenesulfinate, etc.

The amount of the curing agent to be in the bonding composition of the invention may vary within the range capable of attaining the object of the invention. It may fall generally between 0.05 and 20 % by weight, but preferably between 0.1 and 20 % by weight, more preferably between 1 and 10 % by weight of the bonding composition. The method of adding the curing agent to the bonding composition of the invention is not specifically defined, so far as it attains the object of the invention. For example, in case where the bonding composition is of a type for photopolymerization, it may be in a single package containing both a photopolymerization initiator and a polymerization promoter; but where it is of a dual-cure type that contains a thermal polymerization initiator and a polymerization promoter, it may be divided into two or more packages so that a peroxide and an amine or a peroxide and a salt of a sulfinic acid should not be in one and the same package, in order to ensure the storage stability of the composition. Two or more packages separately containing the constituent ingredients of the bonding composition may be blended before use. Apart from it, in case where the bonding composition is applied twice or more to the surface of the object to which it is bonded, another method may be employed. For example, one part of the composition containing a polymerization initiator is first applied to the object, and thereafter another part thereof not containing a polymerization initiator but containing a polymerization promoter alone is applied thereto. This is for ensuring the migration and mixture of the ingredients of the curing agent on the surface of the object to which the bonding composition was applied twice in that manner. In this case, the blend ratio of all the ingredients constituting the bonding composition applied to the object must satisfy the requirement of the invention.

As so mentioned hereinabove, the bonding composition of the invention must contain a curing agent, and the curing agent may be a polymerization initiator alone, or a mixture of a polymerization initiator and a polymerization promoter. Preferably, the bonding composition is curable as rapidly as possible, since the thickness of the uncured surface layer of the composition applied to an object could be thinner. In this case where the rapidly-curable composition is applied onto an object to a predetermined level, its cured layer could all the time enjoy the intended thickness, and the bonding interface could be protected from oxygen that may act thereon as a polymerization retardant. As a result, the bonding interface could be highly polymerized. Accordingly, the polymerization initiator for use in the invention is preferably a photopolymerization initiator, as it enables easy and rapid polymerization of the bonding composition and ensures the storage stability thereof. The photopolymerization initiator gets energy, after having absorbed light, and forms active radicals, and it is generally combined with a polymerization promoter that promotes the radical formation. As the case may be, such a photopolymerization initiator may be additionally combined with a thermal polymerization initiator, and the combination is effective in the invention. Preferably, the bonding composition of the invention is a photopolymerizable one-liquid bonding composition in the form of a single package containing all the necessary constituent ingredients therein. Though not intended, however, the active ingredients of the composition in such single packages may happen to degrade or polymerize while stored. To prevent it, the constituent ingredients of the composition may be divided into two or more parts, and the plural parts are separately packaged and stored in different packages. For their use, the plural parts taken out of the individual packages may be applied to one and the same object in sequence; or they may be blended into one mixture just before use.

Further, the bonding composition of the invention contains a form-retaining agent. In this, the form-retaining layer acts to control the thickness of the bonding layer of the composition. Specifically, it reinforces the bonding layer and ensures the desired thickness of the bonding layer. Accordingly, the polymerization failure in the bonding interface that may be caused by oxygen in air could be prevented, and the bonding interface could be smoothly polymerized and cured to ensure increased bonding strength of the cured bonding layer. In general, the thickness of the bonding layer is controlled to fall between 20 and 300 microns, preferably between 20 and 100 microns or so.

For its morphology, the form-retaining agent to be used herein is not specifically defined, and may be any of granular, tabular, sheet-like, fibrous or porous ones. In view of its handleability, the form-retaining agent is preferably a granular one. To the site to which the bonding composition is applied, a hydrophobic polymerizable composition, for example, a dental restorative composite resin or the like is further applied so as to restore it. Therefore, it is desirable that the form-retaining agent is treated with silane coupling agent or the like so as to improve its compatibility with composite resins, etc. Regarding its size, it is desirable that the form-retaining agent is not so fine to such a degree that it will increase the viscosity of the bonding composition. Microscopically, it is desirable that the particles of the form-retaining agent are large to such a degree that a liquid component could freely move along them but they do not almost move by themselves to increase the viscosity of the bonding composition. For example, the form-retaining agent may have a mean particle size of from 1 to 300 microns, preferably from 3 to 100 microns, more preferably from 5 to 80 microns.

The form-retaining agent is selected from the group consisting of sand balloons, glass balloons, short glass fibers, pieces of hollow glass fibers, glass beads, glass powders, powders of various natural minerals, beads and flakes of various crosslinked polymers, organic/inorganic composite materials containing such inorganic substances and crosslinked polymers. Of these form-retaining agents, preferred are crosslinked polymers as they do not solidify through precipitation or aggregation in the bonding composition and their specific gravity could be nearly the same as that of the bonding composition. The crosslinked polymers may be granular ones to be prepared, for example, through suspension or emulsion copolymerization of the above-mentioned monofunctional (meth)acrylates and polyfunctional (meth)acrylates. The crosslinked polymers may be swollen when mixed with the bonding composition comprising an acid group-having polymerizable compound, a film-forming agent, an organic solvent, etc. In general, the degree of swelling of the crosslinked polymers in those ingredients may be at most 100 %.

The amount of the form-retaining agent that may be in the bonding composition of the invention may fall between 0.5 and 20 % by weight, preferably between 1 and 10 % by weight.

The method to be employed herein for preventing the form-retaining agent from aggregating and solidifying in containers is not specifically defined. For example, hard balls, cylindrical or oval blocks or the like may be put in the container where the bonding composition is housed, and the container is shaken to agitate the contents before the composition is used. In this method, the specific gravity of the form-retaining agent to be used could be negligible with no problem.

As mentioned hereinabove, the preferred embodiment of the blend ratio of the constituent ingredients of the bonding composition of the invention is such that the composition comprises from 1 to 50 % by weight of a (meth)acryloyloxyalkyl phosphate, from 10 to 96 % by weight of a water-soluble film-forming agent, from 1 to 80 % by weight of water, from 0.1 to 20 % by weight of a curing agent, and from 0.5 to 20 % by weight of a form-retaining agent. If desired, additives generally used in ordinary bonding compositions, for example, pigments or dyes for differentiation, viscosity improvers for attaining optimum viscosity and others may be added to the bonding composition of the invention, so far as they do not have any negative influences on the composition. The invention is described more concretely with reference to the following Examples, which, however, are not intended to restrict the scope of the invention. The methods employed in the Examples for measuring the properties of the samples prepared are described below.

### EXAMPLES

### Measurement of pH:

One part by weight of the acid to be measured is mixed with 99 parts by weight of distilled water, and the pH of the resulting mixture is measured with a pH meter (luchi's pH meter).

### Measurement of solubility of calcium salt of acid in water:

The acid to be measured (0.21 mmols for monoacid, 0.105 mmols for diacid) and distilled water (1 ml) are put into a 20 ml test tube, and stirred therein for 10 minutes so that the acid is dissolved in the water. Next, powdery calcium carbonate (0.1 mmols) is fed into the test tube, and stirred at room temperature for 10 minutes so that it is reacted with the acid. The resulting solution is macroscopically checked as to whether it is clear or cloudy. The solubility of the calcium salt in the clear solution is above 0.1 M/L, and the acid is marked "×".

To the cloudy solution in the test tube, 9 ml of distilled water is further added, and stirred at room temperature for 30 minutes. This is again checked macroscopically. When it gives a clear solution in this condition, the solubility of the calcium salt falls between 0.01 and 0.1 M/L, and the acid is marked "Δ". When it is still cloudy in this condition, the solubility of the calcium salt is below 0.01 M/L, and the acid is marked "O". In case where the solubility of the calcium salt of the acid tested is numerically represented, test tubes having a proper capacity are prepared, and the process as above is repeated in them. Salt solutions having different concentrations are prepared, and they are macroscopically checked to quantitatively determine the solubility of the salt. Measurement of solubility of calcium salt of acid in film-forming agent:

The acid to be measured (0.21 mmols for monoacid, 0.105 mmols for diacid) and distilled water (1 ml) are put into a 20 ml test tube, and stirred therein for 10 minutes so that the acid is dissolved in the water. Next, powdery calcium carbonate (0.1 mmols) is fed into the test tube, and stirred at room temperature for 10 minutes so that it is reacted with the acid to give a calcium salt of the acid. Next, the resulting solution is dried in a drier at 60°C for 16 hours to remove water, and the 1 ml of a film-forming agent is added to the resulting residue. This is stirred at room temperature for 30 minutes, and the resulting solution is macroscopically checked as to whether it is clear or cloudy. The solubility of the calcium salt in the clear solution is above 0.1 M/L, and the acid is marked "×".

To the cloudy solution in the test tube, 9 ml of the film-forming agent is further added, and stirred at room temperature for 30 minutes. This is again checked macroscopically. When it gives a clear solution in this condition, the solubility of the calcium salt falls between 0.01 and 0.1 M/L, and the acid is marked "Δ". When it is still cloudy in this condition, the solubility of the calcium salt is below 0.01 M/L, and the acid is marked "O". For easily-available calcium salts of acids, such as calcium chloride, calcium nitrate and others, they are not produced in the manner as above, but commercial products are used to measure their solubility.

### Measurement of bonding strength to the enamel or the dentin of bovine teeth:

A bovine, mandibular central incisor (this is as large as possible.) is used as the object to which the bonding composition to be tested is bonded. Its center that faces the lips is abraded first with waterproof abrasive paper, #320 and finally with #1000 to make a flat surface in enamel portion or in dentin portion. On the flat surface thus-exposed in enamel or dentin, a mending tape (from 3M) with a circular hole (diameter: 3 mm) is stuck in its center, by which the area of the enamel or the dentin to which the bonding composition is applied is defined. 14 bovine tooth samples are prepared for every bonding composition to be tested herein.

The bonding test is effected as follows: In a place where a gentle stream is running (around the inlet of a draft chamber), the bonding composition to be tested is fully applied onto the surface of the exposed enamel or dentin of the bovine tooth sample (its layer formed is about 500 microns thick), and processed as such for 60 seconds. Next, this is exposed to mild air blow so that water and other volatile components are removed to such a degree that the surface of the bonding composition on the dentin is kept glossy. Next, this is exposed to visible rays from a dental light emitter (Ushio Electric's Litel 2) for 20 seconds, and a photopolymerizable dental composite resin, Clearfill Photo SC (from Kuraray, this is for restoring carious teeth) is applied thereover to form a layer of a few mm thick thereon. Then, this is gently pressed against a glass slide superposed thereon. In that condition, this is again exposed to light from the same light emitter as above for 20 seconds so that the composite resin is cured. One end of a stainless steel rod of SUS-304 having a diameter of 5 mm (the other end thereof is holed so that a pin could be fitted therein, and a jig to measure the tensile strength of the bonded sample is fitted to the pin) is attached to the bonded sample vertically to the enamel or dentin surface of the sample, via a dental resin cement (Kuraray's Panavia 21) therebetween. In that condition, the sample is stored in water at 37°C for 2 hours. Thus prepared, seven of 14 samples in one group are subjected to a tensile test, for which is used an Instron universal tester. The cross head speed in the test is 2 mm/min. Five of seven data obtained, excepting the highest and lowest data, are averaged, and the averaged value indicates the bond strength of the samples of the group.

### Measurement of bonding durability:

The remaining seven samples prepared above, which are still in water, are stored as such in a thermostat water tank at 50°C for one month. The thus-stored samples are subjected to the same tensile test as above to measure their bond strength. The data obtained indicate the bonding durability of the samples.

The meanings of the abbreviations used herein are mentioned below.
- HEMA:: hydroxyethyl methacrylate
- HPMA:: hydroxypropyl methacrylate
- GLM:: glycerol monomethacrylate
- PE-200-OH:: monomethacrylate of polyethylene glycol 200
- PE-200-OMe:: methacrylate of methoxypolyethylene glycol 200
- PE-200-OMR:: dimethacrylate of polyethylene glycol 200
- HO-4ED:: monomethacrylate of ethylene glycol tetra- or penta-mer
- 3G:: dimethacrylate of ethylene glycol trimer
- 9G:: dimethacrylate of ethylene glycol nona-mer
- 14G:: dimethacrylate of ethylene glycol tetradeca-mer
- BMHPE:: 1,2-bis(3-methacryloxy-2-hydroxypropoxy)ethane
- PMEP:: phenyl-2-methacryloyloxyethyl phosphate
- 2MEP:: 2-methacryloyloxyethyl phosphate
- 8MOP:: 8-methacryloyloxyoctyl phosphate
- 9MNP:: 9-methacryloyloxynonyl phosphate
- 10MDP:: 10-methacryloyloxydecyl phosphate
- 12MLP:: 12-methacryloyloxydodecyl phosphate
- MRA:: methacrylic acid

- TSA:: p-toluenesulfonic acid
- Bis-GMA:: 2,2-bis[4-(3-methacryloyloxy-2-hydroxypropoxy)phenyl]propane
- MMA:: methyl methacrylate
- TMTA:: trimethylolpropane triacrylate
- UDMA:: [2,2(4),4-trimethylhexamethylenebis(2-carbamoyloxyethyl)] dimethacrylate

### Reference Example (solubility of film-forming agent):

About 1 g of the film-forming agent shown in Table 1 below was metered into a 5 ml glass bottle, and a physiological saline solution of which the weight was the same as that of the film-forming agent was put into the glass bottle, and shaken at room temperature for 20 seconds. The resulting mixture was macroscopically checked as to whether or not it could be a uniform solution. The results obtained are given in Table 1.

**Table 1**

| Film-forming Agent | Solubility | Film-forming Agent | Blend Ratio (wt.%) | Solubility |
|---|---|---|---|---|
| HEMA | dissolved | HEMA/BMHPE | 50/50 | not dissolved |
| HPMA | not dissolved | HEMA/BMHPE | 60/40 | dissolved |
| GLM | dissolved | HEMA/BMHPE | 70/30 | dissolved |
| PE-200-OH | dissolved | HEMA/3G | 90/10 | dissolved |
| PE-200-OMe | not dissolved | HEMA/9G | 23/77 | dissolved |
| PE-200-OMR | not dissolved | HEMA/9G | 50/50 | dissolved |
| HO-4ED | dissolved | GLM/9G | 23/77 | dissolved |
| 9G | not dissolved | GLM/9G | 50/50 | dissolved |
| 14G | dissolved | | | |
| BMHPE | not dissolved | | | |

### Reference Examples 1 and 2, Examples 3 to 6, Comparative Examples 1 to 8:

Acids shown in Table 2 below were used. Bonding compositions comprising 10 parts by weight of the acid, 50 parts by weight of a film-forming agent composed of 90 % by weight of HEMA and 10 % by weight of BMHPE, 40 parts by weight of distilled water, 0.5 parts by weight of camphorquinone, 0.5 parts by weight of N,N-dimethylaminobenzophenone, and 0.5 parts by weight of 2,4,6-trimethylbenzoyldiphenylphosphine oxide were prepared, and their bonding strength and bonding durability were measured according to the methods mentioned above. The.data obtained are given in Table 2. In the bond strength test, the bonded samples of Comparative Examples 1 to 8 peeled at the interface between the bovine tooth and the bonding layer; but in the bonded samples of Examples 1 to 6, several bovine tooth was broken. In particular, almost all the bonded samples of Examples 3 to 6 showed cohesive failure with the bonded bovine tooth broken.

From the results obtained, it is understood that the bonding compositions containing an acid group-having polymerizable compound, of which the solubility of the calcium salt in water and in the film-forming agent in the composition is smaller than 0.01 M/L (this means the calcium salt is insoluble in the two), all have extremely high bonding strength and extremely good bonding durability.

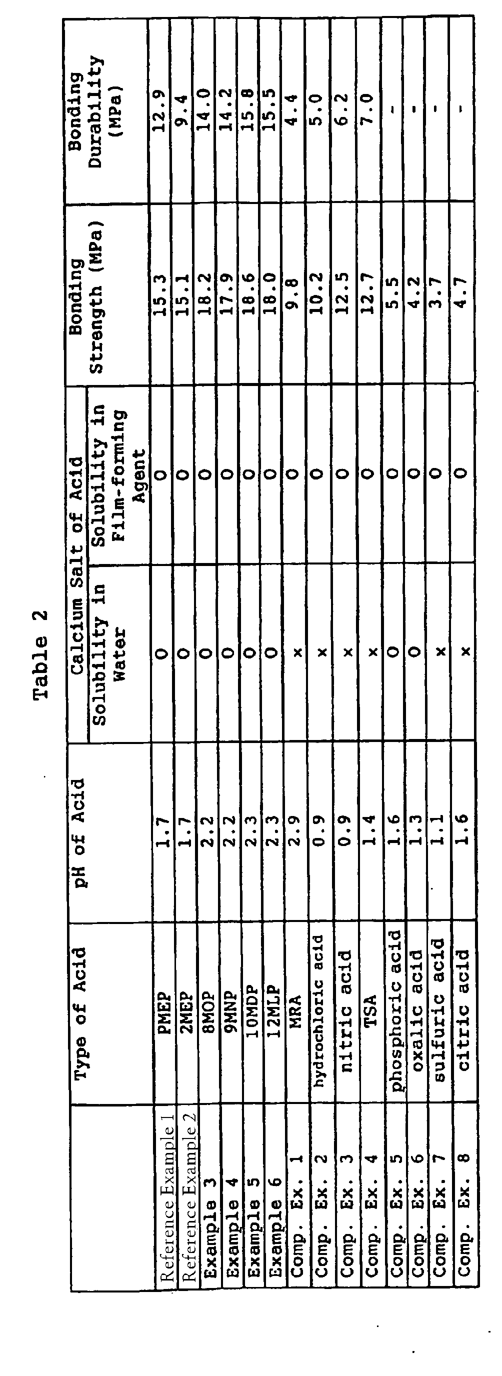

### Examples 7 to 10:

Acids shown in Table 3 below were used. Bonding compositions comprising 5 parts by weight of the acid, 30 parts by weight of a film-forming agent composed of 65 % by weight of HEMA and 30 % by weight of BMHPE and 5% by weight of Bis-GMA, 30 parts by weight of distilled water, 35 parts by weight of ethanol, 0.3 parts by weight of camphorquinone, 0.3 parts by weight of N,N-dimethylaminobenzophenone, 0.3 parts by weight of 2,4,6-trimethylbenzoyldiphenylphosphine oxide, and 2 parts by weight of particles of fused quartz glass (these were prepared by grinding a solid of fused quartz glass into particles of not larger than 25 microns in size, removing fine powder from them through precipitation, and treated them with silane coupling agent, and had a mean particle size of 10 microns) were prepared. Just before use, these were well shaken, and their bonding strength and bonding durability were measured according to the methods mentioned above. The data obtained are given in Table 3. The bonding strength and the bonding durability of all the bonded samples were at least 16 MPa. Almost all of them showed cohesive failure with the bonded bovine tooth broken. From the results obtained, it is understood that the bonding compositions additionally containing a form-retaining agent have more increased bonding strength and more stable bonding durability.

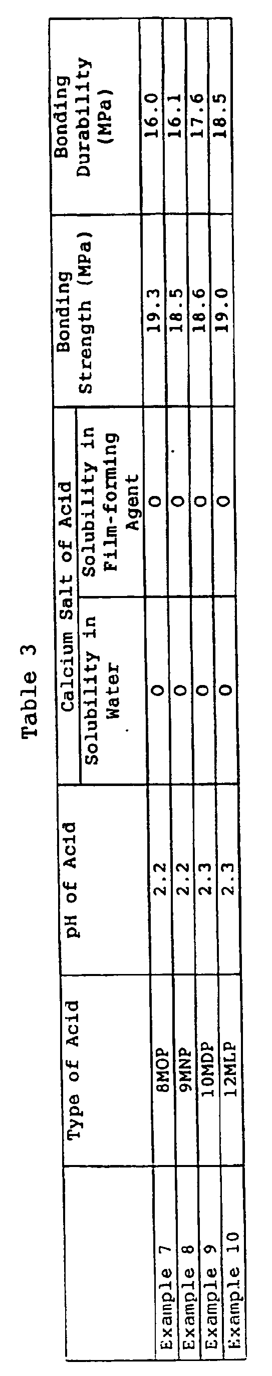

### Examples 11 to 15:

A mixture comprising 10 parts by weight of 10-methacryloyloxydecyl phosphate (the solubility in water of its calcium salt was 0.0003 M/L and the solubility thereof in the film-forming agent used herein was 0.0001 M/L, the latter being obtained through emission spectrometry using a light source of ICP), 30 parts by weight of a film-forming agent composed of 65 % by weight of HEMA, 30 % by weight of BMHPE and 5 % by weight of Bis-GMA, 30 parts by weight of distilled water, 0.3 parts by weight of camphorquinone, 0.3 parts by weight of (2-methacryloyloxy)ethyl N,N-dimethylaminobenzoate, and 30 parts by weight of ethanol was mixed with 5 parts by weight of the form-retaining agent shown in Table 4 to prepare bonding compositions. Just before use, these were well shaken, and their bonding strength and bonding durability were measured according to the methods mentioned above. The data obtained are given in Table 4. From the results obtained, it is understood that the bonding compositions of these Examples all have extremely high bonding strength and extremely good bonding durability, like those of Examples 7 to 10.

**Table 4**

| | Form-retaining Agent | | Bonding Strength (MPa) | Bonding Durability (MPa) |
|---|---|---|---|---|
| | particle size (µm) | Material | | |
| Example 11 | 35 | hollow borosilicate glass 1) | 20.7 | 18.6 |
| Example 12 | 50 | alumina for sand blasting 2) | 21.2 | 18.1 |
| Example 13 | 60 | plastic beads 3) | 17.5 | 17.1 |
| Example 14 | 15 | organic composite filler 4) | 17.1 | 16.0 |
| Example 15 | glass cloth 5) | | 18.8 | 18.0 |

| | | | | |
|---|---|---|---|---|
| 1) Fuji Silicia's glass balloon S-35 | | | | |
| 2) Jelenco's product | | | | |
| 3) Negami Kogyo's TM-150 (MMA/TMTA) | | | | |
| 4) Kuraray's UDMA/3G/Aerosil OX-50 - 30/10/60 | | | | |
| 5) A knitted sheet of glass fibers having a fiber diameter of | | | | |
| 10 µm was cut to give a disc having a diameter of 3 mm. | | | | |

### Example 16:

To 97 g of a mixture comprising 5 parts by weight of 4-methacryloyloxyethoxycarbonylphthalic acid, 45 parts by weight of HEMA, 10 parts by weight of BMHPE, 20 parts by weight of distilled water, 20 parts by weight of ethanol, 0.5 parts by weight of camphorquinone, and 0.3 parts by weight of ethyl N,N-dimethylaminobenzoate, 3 g of silane-treated, hollow borosilicate glass of 35 microns in size were added, and well mixed to prepare a bonding composition. Its bonding strength and bonding durability were measured according to the methods mentioned above, and were 16.7 MPa and 15.0 MPa, respectively.

### Comparative Examples 9 to 12:

Bonding compositions were prepared in the same manner as in Example 5, except that the acid only was omitted (Comparative Example 9), the film-forming agent only was omitted (Comparative Example 10), water only was omitted (Comparative Example 11), and the curing agent only was omitted (Comparative Example 12). Their bonding strength and bonding durability were measured according to the same methods as in Example 5. The data obtained are given in Table 5. From these, it is understood that the composition not containing the acid is not adhesive and that the compositions not containing any of the film-forming agent, water and the curing agent have poor bonding strength and poor bonding durability.

**Table 5**

| | Bonding Strength (MPa) | Bonding Durability (MPa) |
|---|---|---|
| Comparative Example 9 | 0 | 0 |
| Comparative Example 10 | 6.5 | 4.5 |
| Comparative Example 11 | 9.0 | 3.6 |
| Comparative Example 12 | 12.2 | 8.0 |

### Example 17, Comparative Examples 13, 14:

The bonding strength and the bonding durability of the bonding composition of Example 9 to the enamel and the dentin of bovine teeth that had been etched with an acid were compared with those to the enamel and the dentin of bovine teeth not having been etched with an acid. For the former, bovine teeth were etched in the manner mentioned below. The data obtained are given in Table 6. (For reference, the data of Example 9 are also given in Table 6.) To etch them, a commercially-available, phosphoric acid etchant (Kuraray's K-etchant; aqueous 38 % phosphoric acid solution) was applied to the surface of each bovine tooth to which the bonding composition is to be applied, and kept at room temperature for 30 seconds. Next, the processed surface of each bovine tooth was washed with running water for 30 seconds, and then exposed to clean compressed air by which water was blown off and the surface was dried. From the data, it is understood that the bonding strength and the bonding durability of the bonding composition of the invention to the non-etched objects are on the same level as those to the phosphoric acid-etched objects. This supports the excellent bonding ability of the bonding composition of the invention even to non-etched objects.

**Table 6**

| | Condition for Treatment | Bonding Strength (MPa) | Bonding Durability (MPa) |
|---|---|---|---|
| Example 9 | Bovine dentin not etched | 18.6 | 17.6 |
| Comp. Ex. 13 | Bovine dentin etched | 18.3 | 17.0 |
| Example 17 | Bovine enamel not etched | 21.2 | 17.7 |
| Comp. Ex. 14 | Bovine enamel etched | 19.0 | 17.8 |

### Example 18:

According to the method employed hereinabove for preparing tooth samples for measurement of the bonding strength of bonding compositions thereto, a bovine tooth was polished to prepare a tooth sample with the dentin exposed. A part of the polished dentin surface was masked with a varnish (Kuraray's Protect Varnish); and the bonding composition of Example 5 was applied to the sample to broadly cover both the masked and non-masked area, then left as such for 60 seconds, and thereafter exposed to air streams to evaporate the volatile components of the composition. Next, a polymerizable resin (Kuraray's pit and fissure sealant, Teethmate F1) was applied thereover to form a thick film thereon, and exposed to light to cure the resin. The thus-processed bovine tooth sample was cut into two parts that contained both masked part and non-masked part, and polished. The cross section of each part was observed with an electronic microscope (Hitachi's S-510) . As a result, a level difference of about 0.5 microns was found between the masked part and the non-masked part. This indicates that the bonding composition dissolved the dentin of the tooth sample.

On the other hand, the bonding composition of Example 5 was applied to the surface of the polished bovine tooth sample with the dentin exposed to form a thick film thereon. After left as such for 1 minute, the crude product of the bonding composition was collected from the sample by the use of a microsyringe, and its pH was measured. The pH of the collected product was higher by 0.7 than that of the unused composition. From this, it is understood that the acid ingredient of the bonding composition reacted with the tooth and formed its calcium salt, whereby the product of the composition was neutralized. This supports the self-etching ability of the bonding composition of the invention.

### Example 19, Comparative Example 15:

Two drops of the bonding composition of Example 1 were put into a test tube, and the solvent (water) was removed by air blowing thereto. This was exposed to light from the same light emitter as that used in Example 1, and immediately it gave heat of polymerization. Thus, it cured extremely rapidly. For comparison, the two-liquid type bonding composition of Example 2 of Japanese Patent Laid-Open No. 45510/1985 was tested in the same manner. Briefly, one drop of liquid D and one drop of liquid B were mixed in a test tube, and the solvent was removed by air blowing thereto. While left at 23°C, the test tube was monitored for the temperature change by feeling it with fingers at regular intervals of 10 seconds. After 30 to 40 seconds, it gave heat, and began to polymerize.

As described in detail hereinabove with reference to its embodiments, the bonding composition of the invention comprises an acid group-having polymerizable compound, a water-soluble film-forming agent, water and a curing agent, in which the calcium salt of the acid is insoluble in water and in the film-forming agent, and the film-forming agent is a polymerizable compound and is miscible with a physiological saline solution. Accordingly, the bonding composition has the function of self-etchability by itself, and, when used in dental treatment, it does not require any pre-treatment including etching treatment with phosphoric acid or the like and priming treatment of teeth. To the teeth to which it is applied, the bonding composition has high bonding strength and good bonding durability, and its bonding strength stably lasts for a long period of time

In case where a form-retaining agent is added to the bonding composition, the strength of the bonding layer of the composition is much increased, and, in addition, the bonding layer could ensure the desired thickness. Accordingly, the polymerization failure in the bonding interface that may be caused by oxygen in air could be prevented, and the bonding interface could be smoothly polymerized and cured to ensure increased and stable bonding strength of the cured bonding layer.

In addition, as so mentioned above, the dental treatment with the bonding composition of the invention does not require etching treatment or priming treatment. Therefore, the bonding composition reduces the dentists' labor for treatment therewith and shortens the time for it. In addition, the time for which the patients must open their mouth could be shortened, and the patients' pain could be reduced. Thus, the bonding composition of the invention is useful in dental treatment.

## Claims

1. A bonding composition suitable to tooth tissue, which comprises a mixture of a (meth)acryloyloxyalkyl phosphate in which the number of the carbon atoms constituting the alkyl group falls between 6 and 24, a water-soluble film-forming agent, water, a form-retaining agent selected from the group consisting of sand balloons, glass balloons, short glass fibers, pieces of hollow glass fibers, glass beads, glass powders, powders of natural minerals, boads and flakes of crosslinked polymers and organic / inorganic composite materials containing said inorganic substances or crosslinked polymers, and a curing agent, and in which the calcium salt of the acid is insoluble in water, and the film-forming agent is a polymerizable compound and is miscible with a physiological saline solution.

2. The bonding composition as claimed in claim 1, in which the pH value of an aqueous 1 wt.% solution of the (meth)acryloyloxyalkyl phosphate falls 1.8 2.5.

3. The bonding composition as claimed in anyone of claims 1 to 2, in which the film-forming agent is a polymerizable compound comprising 2-hydroxyethyl methacrylate as the essential ingredient. 3

4. The bonding composition as claimed in anyone of claims 1 to 3, in which the curing agent is a photopolymerization initiator or a mixture of a photopolymerization initiator and a polymerization promoter.

5. The bonding composition as claimed in anyone of claims 1 to 4, which is one-liquid type. rapidly-curable bonding composition.

6. The bonding composition as claimed in anyone of claims 1 to 5, in which the form-retaining agent is crosslinked polymer particles.

## Patentansprüche

1. Klebezusammensetzung, geeignet für Zahngewebe, welches ein Gemisch von einem (Meth)acryloyloxyalkylphosphat, worin die Anzahl der Kohlenstoffatome, welche den Alkylrest konstituieren, zwischen 6 und 24 fällt, von einem wasserlöslichen Filmbildungsmittel, Wasser, einem Formhaltemittel, ausgewählt aus der Gruppe, bestehend aus Sandkugeln, Glaskugeln, kurzen Glasfasern, Stücken von Hohlglasfasern, Glasperlen, Glaspulver, Pulvern von natürlichen Mineralien, Kügelchen und Flakes von vernetzten Polymeren und organischen/anorganischen Verbundmaterialien, welche die anorganischen Substanzen oder vernetzte Polymere enthalten, und einem Härtemittel, worin das Calciumsalz der Säure in Wasser unlöslich ist, und das Filmbildungsmittel eine polymerisierbare Verbindung ist und mit einer physiologischen Kochsalzlösung mischbar ist.

2. Klebezusammensetzung, wie in Anspruch 1 beansprucht, worin der pH-Wert einer wäßrigen 1 Gew.-%-igen Lösung des (Meth)acryloyloxyalkylphosphats zwischen 1,8 und 2,5 fällt.

3. Klebezusammensetzung, wie in einem der Ansprüche 1 bis 2 beansprucht, worin das Filmbildungsmittel eine polymerisierbare Verbindung ist, welche 2-Hydroxyethylmethacrylat als den wesentlichen Bestandteil umfaßt.

4. Klebezusammensetzung, wie in einem der Ansprüche 1 bis 3 beansprucht, worin das Härtungsmittel ein Photopolymerisationsinitiator oder ein Gemisch eines Polymerisationsinitiators und eines Polymerisationspromoters ist.

5. Klebezusammensetzung, wie in einem der Ansprüche 1 bis 4 beansprucht, welches eine schnell-härtbare Klebezusammensetzung vom Einzeiflüssigkeitstyp ist.

6. Klebezusammensetzung, wie in einem der Ansprüche 1 bis 5 beansprucht, worin das Formhaltemittel vernetzte Polymerteilchen ist.

## Revendications

1. Composition adhésive appropriée pour le tissu dentaire, comprenant un mélange d'un phosphate de (méth)acryloyloxyalkyle dans lequel le nombre d'atomes de carbone constituant le groupe alkyle est compris entre 6 et 24, d'un agent filmogène soluble dans l'eau, d'eau, d'un agent maintenant la forme choisi dans l'ensemble constitué de ballons de sable, de ballons de verre, de fibres de verre courtes, de morceaux de fibres de verre creuses, de perles de verre, de poudres de verre, de poudres de minéraux naturel, de perles et de flocons de polymères réticulés et de matériaux composites organiques/inorganiques contenant lesdites substances inorganiques ou polymères réticulés, et d'un agent de durcissement, et dans laquelle le sel de calcium de l'acide est insoluble dans l'eau, et l'agent filmogène est un composé polymérisable et est miscible avec une solution saline physiologique.

2. Composition adhésive selon la revendication 1, dans laquelle la valeur de pH d'une solution aqueuse à 1 % en poids du phosphate de (méth)acryloyloxyalkyle est comprise entre 1,8 et 2,5.

3. Composition adhésive selon l'une quelconque des revendications 1 à 2, dans laquelle l'agent filmogène est un composé polymérisable comprenant du méthacrylate de 2-hydroxyéthyle en tant qu'ingrédient essentiel.

4. Composition adhésive selon l'une quelconque des revendications 1 à 3, dans laquelle l'agent de durcissement est un initiateur de photopolymérisation ou un mélange d'un initiateur de photopolymérisation et d'un promoteur de polymérisation.

5. Composition adhésive selon l'une quelconque des revendications 1 à 4, qui est une composition adhésive de type à un liquide et durcissant rapidement.

6. Composition adhésive selon l'une quelconque des revendications 1 à 5, dans laquelle l'agent maintenant la forme est constitué de particules de polymère réticulé.
